# EUROPEAN PATENT SPECIFICATION

(11) **EP 4 125 588 B1**
(45) Date of publication and mention of the grant of the patent: **21.10.2026**
(21) Application number: 21722740.4
(22) Date of filing: 22.03.2021
(51) Int. Cl.: A61B 5/20, A61B 5/00, A61B 5/103, A61B 5/145, A61B 10/00

(54) **DISPOSABLE INDICATOR COMPONENT FOR MEASURING ANALYTE CONCENTRATION IN BODILY FLUIDS**
EINWEG-INDIKATORKOMPONENTE ZUR MESSUNG DER ANALYTKONZENTRATION IN KÖRPERFLÜSSIGKEITEN
COMPOSANT INDICATEUR JETABLE POUR MESURER LA CONCENTRATION D'UN ANALYTE DANS DES FLUIDES CORPORELS

(30) Priority: 23.03.2020 US 202062993137 P; 18.03.2021 US 202117205460
(43) Date of publication of application: 08.02.2023
(73) Proprietor: Kenvue Brands LLC, Summit, NJ 07901 (US)
(72) Inventor: BINNER, Curt, Skillman, New Jersey 08558 (US); MELLINGER, Justin, Skillman, New Jersey 08558 (US); PAUNESCU, Alexandru, Skillman, New Jersey 08558 (US)
(74) Representative: Carpmaels & Ransford LLP
(86) International application number: PCT/US2021/070294
(87) International publication number: WO 2021/195656

(56) References cited:
- WO-A2-2019/140224
- US-A1- 2017 252 225

## Description

### BACKGROUND OF THE INVENTION

### Field of the Invention

The present invention relates to systems which measure changes in the concentration of analytes in bodily fluids. More particularly, this invention relates to systems which are used to measure the concentration of analytes in urine over time and methods to measure these analytes and detect early onset disease states in the human body.

### Description of Related Art

US 2017/0252225 A1 relates to an absorbent article for personal hygiene. The absorbent article absorbent article can include at least one property changing indicator. A detector device is also provided that includes at least one sensor. The sensor is adapted to detect the property change of the property change indicator in the absorbent article. The property changing indicator may include an optical property indicator such as a color change indicator and the sensor may include an optical sensor such as a color sensor.

WO 2019/140224 A2 relates to a system with an analyzer device in fluid communication with a sample of a bodily fluid which is configured to chemically or electrochemically convert at least a portion of ammonium (NH₄⁺) contained within the bodily fluid into ammonia (NH₃) and dispel the converted ammonia (NH₃) into a gas sensing chamber. An ammonia (NH₃) sensor located within the gas sensing chamber in conjunction with a processor can quantify an amount of ammonia (NH₃) present in the gas sensing chamber in relation to the total ammonia of the bodily fluid.

The analytes found in bodily fluids such as urine or sweat potentially carries evidence of developing metabolic system problems. There is a desire for people in and out of the medical establishment to track and analyze changes in the concentration of analytes in bodily fluids over time.

Currently, people and physicians rely on visible symptoms to diagnose systemic metabolic problems. This often prompts physicians to do urine analysis or blood tests to determine presence or concentrations of various analytes in these bodily fluids. So, in today's practice, test such as urine analysis is most often used to confirm symptom-based diagnosis, rather than as initial identification of disease. Some conditions, like diabetic ketoacidosis, show visible symptoms only when a person's condition may already warrant an emergency visit to a physician. Other conditions, like urinary tract infection, may not show visible symptoms and result in renal scarring, which may not manifest itself in health problems until many years later.

Non-invasively measuring the analyte concentration in urine content is also ideally suited for epidemiological studies to rapidly identify problems prevalent in specific geographies. Difficulty of sample collection, however, prevents acceleration of research in this area.

Absorbent articles such as diapers exist with embedded sensors that are only capable of detecting wetness. Often, they transmit that information to a receiving system. The receiving system then alerts a caregiver of a one-time event. These wetness detection systems do not perform a diagnosis.

Some existing diagnostic systems rely on urinalysis strips being dipped into a urine sample and are manually or automatically read by an imaging device or cell phone. Other diagnostic systems rely on urinalysis strips mounted to the exterior surface of an absorbent article, and, once wet, are manually or automatically read by an imaging device or cell phone. In either case, data from present readings can be compared with those of both past and future readings.

In either approach, the reading of the urinalysis strips is performed at a point in time after the strips have become wet with urine. Many of the chemicals used in the test strips are sensitive to time, temperature, degree of wetness, etc. of exposure. So, accurate and repeatable readings are difficult to obtain.

In summary, analytes found in bodily fluid may evidence of developing metabolic system problems. There is a desire for to track and analyze changes in the concentration of analytes in bodily fluids such as urine over time. However, for the data to be valuable, the readings must be accurate and repeatable.

### BRIEF SUMMARY OF THE INVENTION

The invention is set out in the appended set of claims.

We have developed a novel and useful disposable indicator component for use in a system for measuring analyte concentration in a bodily fluid. The disposable indicator component includes an indicator zone comprising at least one colorimetric analyte sensing element, and a coupler for coupling the indicator component to a component having at least one spectrophotometer contained within a housing.

A disposable indicator component for use with a handheld analyzer can include a first flexible web layer; a fluid transport layer adjacent the first flexible web layer; and a fluid impervious envelope surrounding the indicator zone adjacent the fluid transport layer. The first flexible web layer, fluid transport layer, and the fluid impervious envelope are stacked in order and secured together, and the indicator zone comprises at least two colorimetric analyte sensing elements. In addition, the fluid impervious envelope has a discrete pocket arranged on configured to contain each of the at least two colorimetric analyte sensing elements and each pocket has a unique aperture in fluid communication with the fluid transport layer, and the fluid transport layer is arranged and configured to inhibit fluid transport between apertures in the fluid impervious envelope.

### BRIEF DESCRIPTION OF THE SEVERAL VIEWS OF THE DRAWINGS

FIG. 1 is a top perspective view of a system for measuring analyte concentration in an absorbent article of the present invention;
FIG. 2 is an exploded view of the system of FIG. 1;
FIG. 3 is a cross-sectional view of the indicator component of the system of FIG. 1;
FIG. 4 is a top view of the durable component of the system of FIG. 1;
FIG. 5 is a cross-sectional view of the durable component of along the 5-5 plane of FIG. 4;
FIG. 6 is a cross-sectional view of the spectrophotometer portion of the durable component of FIG. 5;
FIG. 7 is a top view of the moisture sensor element of the indicator component of the system for measuring analyte concentration in an absorbent article as a moisture front crosses the element;
FIG. 8 is a capacitance versus time plot as a moisture front crosses the moisture sensor element of the indicator component of the system for measuring analyte concentration;
FIG. 9 is a top perspective view of a system for measuring analyte concentration of the present invention;
FIG. 10 is a bottom perspective view of the system for measuring analyte concentration of FIG. 9;
FIG. 11 is an exploded view of the indicator component of the system of FIGs. 9 and 10;
FIG. 12 is a top perspective view of the fluid impervious envelope encapsulating colorimetric analyte sensing elements of the indicator component of FIG 11;
FIG. 13 is a top view of the fluid impervious envelope encapsulating colorimetric analyte sensing elements of the indicator component FIG. 11;
FIG. 14 is a top view of partially assembled indicator component of FIG. 11;
FIG. 15 is a bottom view of partially assembled indicator component of the FIG. 11;
FIG. 16 is a top perspective view the durable component of the system of FIGs. 9 and 10;
FIG. 17 is a top view of the durable component of the system of FIGs. 9 and 10;
FIG. 18 is a top perspective view of a system for measuring analyte concentration in a bodily fluid of the present invention;
FIG. 19 is a top perspective view of the indicator component of the system of FIG. 18; and
FIG. 20 is a partially exploded view of the system of FIG. 18.

### DETAILED DESCRIPTION OF THE INVENTION

The present invention relates to systems for use in absorbent articles which measure changes in the concentration of analytes in bodily fluids such as urine over time. The system of the invention may be used in methods to measure the concentration of analytes in bodily fluids over time, as well as methods to use these analyte measurements over time to detect early onset disease states in the human body.

The presently disclosed subject matter will now be described more fully hereinafter with reference to the accompanying drawings and examples. The presently disclosed subject matter can, however, be embodied in different forms and should not be construed as limited to any specific examples set forth herein and is to be accorded the widest scope consistent with the features described herein. Rather, any specific examples are provided so that this disclosure will be thorough and complete and will fully convey the scope of the invention to those skilled in the art to which the invention belongs. It is believed that one skilled in the art can, based upon the description herein, utilize the present invention to its fullest extent.

Unless otherwise defined, all technical and scientific terms used herein have the same meaning as commonly understood by one of ordinary skill in the art to which this presently described subject matter belongs.

The present invention relates to systems to enable monitoring of analyte concentration in an absorbent article. The systems also allow statistical analysis and determination of changes in the health state by the collection of multiple data points over time, which may be evidence of developing metabolic system problems. Other data such as medical and family history as well as current variables such as age, temperature, and/or other current markers may be used to supplement trend and statistical analysis.

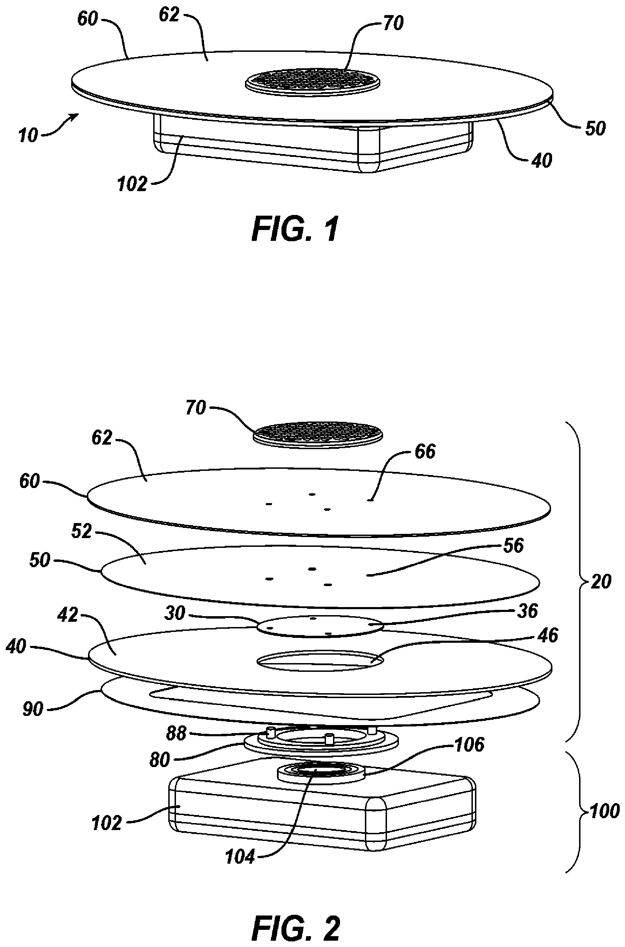

FIGS. 1 to 6 show an apparatus or system **10** for measuring analyte concentration in an absorbent article. System **10** has an indicator component **20** and a durable component **100.** FIG. 1 is a top perspective view of system **10** when full assembled, while FIG. 2 is an exploded view of system **10.**

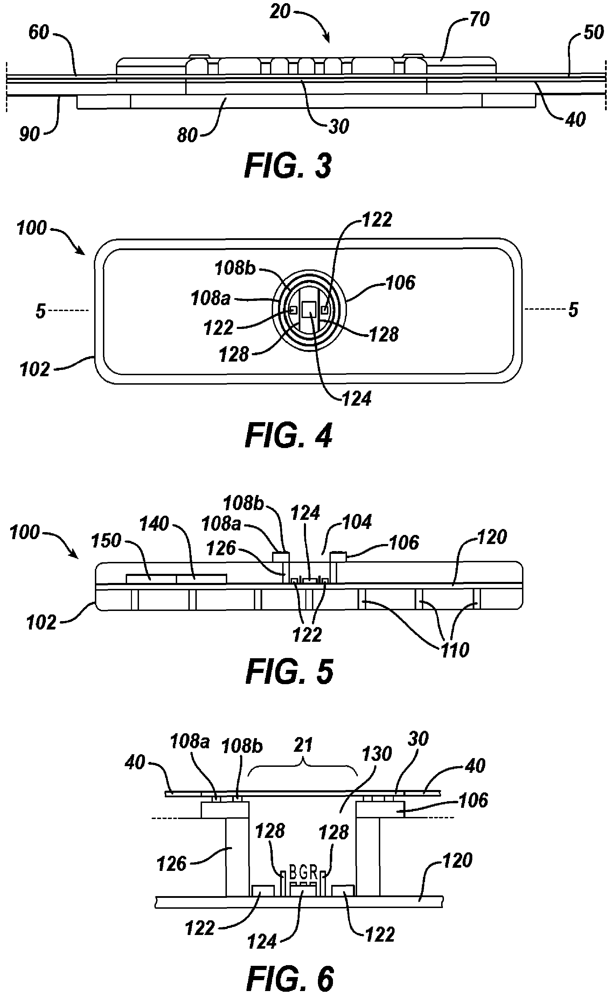

Indicator component **20** is shown in exploded view in FIG. 2, and in cross-sectional view in FIG. 3. Indicator component **20** includes an indicator zone **21** that has a colorimetric analyte sensing element **30** which may be disposed in an optional second flexible web **40,** a fluid transport layer **50,** an optional first flexible web **60,** an optional top plate **70,** a coupler shown here as holding plate **80,** and an adhesive layer **90.** The indicator component **20** is preferably disposable.

Colorimetric analyte sensing element **30** has perforations **36** and is disposed in aperture **46** of second flexible web **40.** In some embodiments, colorimetric analyte sensing element **30** is a reagent impregnated matrix designed to produce a visual indication of the presence of a preselected analyte in sample produced by the wearer of system **10.** Chemistries and methods of detecting analytes by producing a visual indication are well known in the art. In some embodiments, the preselected analyte measured by system **10** may be, glucose, ketone, bilirubin, blood, pH, protein, urobilinogen, nitrite, leukocytes, and/or creatinine, among others.

For example, the absorbent article may be a diaper, the fluid being tested may be urine and the preselected analyte measured by system **10** may be glucose. Glycosuria, or glucose in the urine, is the presence of higher than normal levels of sugar in the urine and may be due to complications with one's kidneys or diabetes. Some of the most common causes of glucose in the urine include: diabetes mellitus, hyperthyroidism, benign glycosuria, liver cirrhosis, or a high sugar diet. In addition, in some embodiments, one of ordinary skill in the art will recognize that choosing appropriate biosensor(s) capable of converting a preferred biomarker into a calorimetrically readable result could be used in genomics, transcriptomics, metabolomics and proteomics as well to determine the presence of inflammatory biomarkers that are present in urine.

As mentioned, colorimetric analyte sensing element **30** is disposed in opening **46** of second flexible web **40** and is in fluid communication with fluid transport layer **50.** Fluid transport layer **50,** in turn, is in fluid communication with first flexible web **60.** Second flexible web **40** has a first side **42,** and is made of non-absorbing material, such as a polyethylene foam. Fluid transport layer **50** has a first side **52,** and perforations **56,** and is made of wicking material, such as fabric or paper, that is effective in spreading and transporting fluid via capillary action. First flexible web **60** has a first side **62,** and perforations **66,** and is made of a non-absorbing apertured film, such as a polyethylene mesh.

Second flexible web **40,** fluid transport layer **50,** and first flexible web **60** are designed to aid in the transport of fluid to colorimetric analyte sensing element **30.** In use, fluid from the absorbable article first contacts first side **62** of first flexible web **60.** Since first flexible web **60** is a non-absorbing apertured film, fluid passes through first flexible web **60** and contacts first side **52** of fluid transport layer **50.** The fluid then permeates throughout fluid transport layer **50.** The fluid will contact first side **42** of second flexible web **40.** But, since second flexible web **40** is made of non-absorbing material, fluid in transport layer **50** does not penetrate second flexible web **40.** Finally, the fluid in transport layer **50** comes into contact with colorimetric analyte sensing element **30.**

Sensing element **30** disposed in second flexible web **40,** fluid transport layer **50,** and first flexible web **60** are stacked, as shown in FIGS. 1 to 3, and are held together by top plate **70** and holding plate **80.** Holding plate **80** has pins **88.** Pins **88** sequentially pass through perforations **36** of colorimetric analyte sensing element **30,** perforations **56** of fluid transport layer **50,** and perforations **66** of first flexible web **60.** Though not shown, top plate **70** has blind holes in which pins **88** are disposed. A friction fit between top plate **70** blind holes and pins **88** hold the components of indicator component **20** together. Alternative assemblies may be held together by other interactions, such as snap fit, ultrasonic weld, heat weld, other mechanical fasteners, and the like.

The top plate and holding plate are arranged and configured to provide a predetermined spacing to accommodate indicator component layers with predetermined fluid transport capacity to the indicator zone. This provides a more controlled delivery of bodily fluid to the indicator zone and the associated timing between the bodily fluid arriving at the indicator zone and the colorimetric measurement, described in more detail, below.

Top plate **70** may have channels on the side facing first side **62** of first flexible web **60.** The channel may help direct fluid from the absorbent article to the first side **62** of first flexible web **60.**

Durable component **100** is shown in exploded top perspective view in FIG. 2, in top view in FIG. 4, in cross-sectional view in FIG. 5, and in enlarged cross-sectional view in FIG. 6. Durable component **100** has a housing **102** with a window **104.** A spectrophotometer is disposed in housing **102.** The components of the spectrophotometer include light sources **122** and photodetector **124.** The spectrophotometer is adjacent to and in optical communication with window **104.** This allows spectrophotometer to be in optical communication with the colorimetric analyte sensing element **30** of indicator component **20.**

As shown in FIG. 2, spectrophotometer includes two light sources **122** and one photodetector **124.** If desired, a spectrophotometer may include at least one or more light sources **122** and at least one photodetector **124,** for example, at least two or more light sources **122** and at least two or more photodetectors **124.**

FIG. 2 also shows male connector protrusion **106** surrounding window **104** on housing **102.** Male connector protrusion **106** allows durable component **100** to be releasably attached to indicator component **20.**

FIG. 4 is a top view of durable component **100** of system **10.** Conductive strips **108a** and **108b** are disposed on the top surface of male connector protrusion **106,** and, as described below, act as a moisture sensor, arranged and configured to communicate the presence of moisture in colorimetric analyte sensing element **30** to the computing system disposed in durable component **100.**

FIGs. 4 and 5 also show two light sources **122** and photodetector **124** linearly arranged in system **10** and evenly spaced. Even spacing may be achieved in other ways, such as multiple two light sources **122** evenly disposed in a square or circular arrangement around photodetector **124.**

FIGs. 5 and 6 are cross-sectional views of durable component **100.** FIG. 5 shows housing **102,** window **104,** male connector protrusion **106,** conductive strips **108a** and **108b,** and printed circuit board (PCB) **120.** FIG. 6 is an enlargement of the region of durable component **100** housing the components of a spectrophotometer.

PCB **120** mechanically supports and electrically connects electronic components using conductive tracks, pads and other features etched from copper sheets laminated onto a non-conductive substrate. Components (e.g. capacitors, resistors, controllers, power sources, light sources, detectors) are generally soldered on PCB **120.** PCB **120** has a computing system **140** having one or more processors and a memory, as well as means for electronic communication **150** to send the results of analyses to data processing systems that are external to system **10.** Data processing systems that may be used include at least one external device including server computers, client computers, and handheld devices such as cellphones.

FIG. 5 shows PCB **120** supported within the housing **102** of durable component **100** by means of support brackets **110.** In other embodiments, PCB **120** may be attached directly to the inner surface of housing **102.**

FIG. 6 is an enlargement of the region of durable component **100** housing the components of a spectrophotometer. Light sources **122** and photodetector **124,** which are the components of the spectrophotometer, are disposed on the surface of PCB **120.** They are shielded from ambient light by shield **126,** shown as a cylindrical ring, with the two ends terminating on the surface of PCB **120** and the inner surface of the housing **102** of durable component **100.** Skirts **128** are attached to the surface of PCB **120** and serve to optically isolate photodetector **124** from light sources **122.** So, in operation, light which emanates from light sources **122** cannot impinge on photodetector **124** without having reflected off of colorimetric analyte sensing element **30.**

Alternatively, lenses can be placed over light sources **122** so that in operation light which emanates from light sources **122** cannot impinge on photodetector **124** without having reflected off of colorimetric analyte sensing element **30.** Potting materials can also be used to focus the light from light sources **122** at colorimetric analyte sensing element **30.**

FIG. 6 also shows light chamber **130.** Light chamber **130** is the volume enclosed by the surface of PCB **120,** ambient light by shield **126,** male connector protrusion **106,** conductive strip **108b,** and colorimetric analyte sensing element **30.** Indicator zone **21** is the area of indicator component **20** where colorimetric analyte sensing element **30** is exposed to light sources **122.**

Though two light sources **122** are apparent in FIGs. 5 and 6, durable component **100** may have multiple light sources **122,** for example four light sources evenly spaced about the device. Light sources **122** may be light-emitting diodes (LEDs), a semiconductor light source that emits light when current flows through it. LEDs have many advantages over incandescent light sources, including lower energy consumption, longer lifetime, improved physical robustness, smaller size, and faster switching. In the embodiment discussed here, light sources **122** are RGB LEDs. Mixing red, green, and blue sources can produce white light with proper blending of the colors. In addition, the colors emanating from RGB LEDs may be monochromatic, allowing data to be obtained in narrow wavelength regions.

Photodetector **124** is also called a photosensor. Photodetectors are sensors of light or other electromagnetic radiation. A photodetector has a p-n junction that converts light photons into current. The absorbed photons make electron-hole pairs in the depletion region. In some embodiments, photodetector **124** can measure the amount of white light received. In the embodiment discussed here, photodetector **124** specifically measures the red, green, and blue light, allowing data to be obtained in narrow wavelength regions (Note in FIG 6 the "R", "G" and "B" above photodetector **124**).

In system **10** for measuring analyte concentration in an absorbent article, light sources **122** emit light in narrow red, green, and blue wavelengths. The emitted light waves reflect off of colorimetric analyte sensing element **30.** The reflected light is then measure by photodetector **124.** In this embodiment, sources **122** sequentially emit red light, green light, and blue light, allowing for the near simultaneous collection of three data points. In other embodiments, the sequence of emitted red light, green light, and blue light may vary.

The components of the spectrophotometer may be coated with a protective material. The protective material keeps the moisture from the colorimetric analyte sensing element **30** from contacting, and potentially damaging, the components of the spectrophotometer.

Indicator component **20** is arranged and configured for releasable attachment to the durable component **100.** When assembled, colorimetric analyte sensing element **30** is disposed adjacent to and in optical communication with window **104** and the elements of the spectrophotometer.

FIGs. 4 to 6 also show conductive strips **108a** and **108b** that are disposed on the top surface of male connector protrusion **106.** Conductive strips **108a** and **108b** act as a moisture sensor in system **10** and are arranged and configured to communicate the presence of moisture in colorimetric analyte sensing element **30** to the computing system disposed in durable component **100.** In turn, the computing system disposed in durable component **100** is operatively connected to the moisture sensor and the components of the spectrophotometer.

As shown in FIG. 6, conductive strips **108a** and **108b** are adjacent to colorimetric analyte sensing element **30.** When moisture impinges on colorimetric analyte sensing element **30,** it will also contact portions of conductive strips **108a** and **108b.**

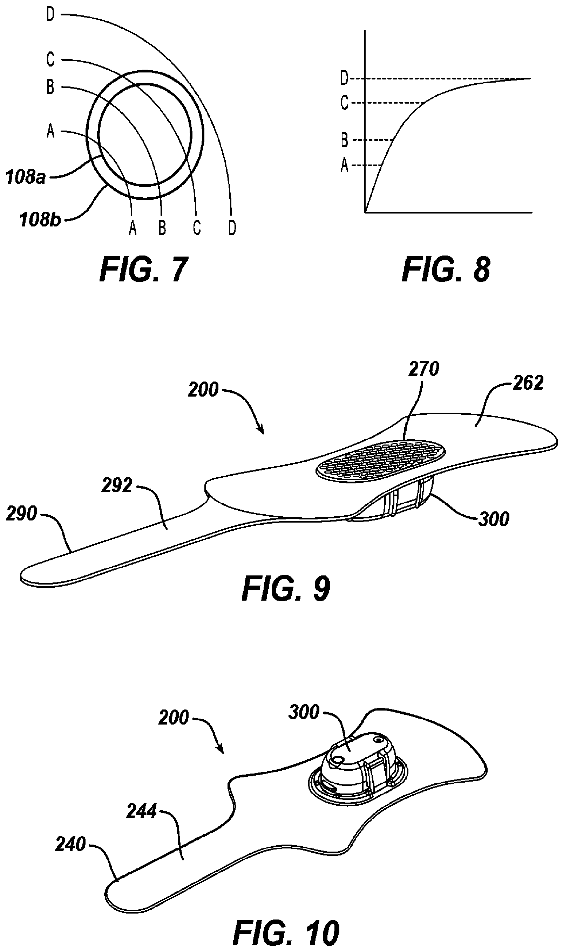

FIGs. 7 and 8 describe the function of conductive strips **108a** and **108b** in the moisture sensor in system **10.** FIG. 7 is a top view of conductive strips **108a** and **108b** at several time points during the progression of a moisture front across the strips. The progression of the front is shown as A-A, B-B, C-C and D-D. At time point A-A, the moisture front has progressed partially across conductive strips **108a** and **108b.** Further progression across strips **108a** and **108b** are shown as time points B-B and C-C, while D-D shows a time point where the moisture front has fully crossed strips **108a** and **108b.**

FIG. 8 shows an example of the change in an electrical property between strips **108a** and **108b** as the moisture front progresses across the strips. In this embodiment, FIG. 8 shows a capacitance versus time plot as a moisture front crosses strips **108a** and **108b.** Line A on FIG. 8 corresponds to time point A-A, where the moisture front has progressed partially across conductive strips **108a** and **108b.** Capacitance is shown to increase to line B and then line C as time points B-B and C-C show further progression across strips **108a** and **108b.** Finally, line D, where capacitance is shown to level of corresponds to time point D-D, where the moisture front has fully crossed strips **108a** and **108b.** At point D-D, colorimetric analyte sensing element **30** has been fully saturated with moisture.

Though capacitance is discussed in this embodiment, other electrical properties, such as resistance, will also change as the moisture front progresses across strips **108a** and **108b.**

The moisture sensing system described above allows the spectrophotometer to perform its reading of the emitted light waves reflect off of colorimetric analyte sensing element **30** at a point in time after the strips have become wet with moisture. This solves the issue of chemicals used in the test strips are sensitive to time, temperature, and degree of wetness, allowing accurate and repeatable readings are to be obtained.

In a preferred embodiment, the plurality of light sources **122** are four narrow beam LEDs spaced about the photodetector **124.** Therefore, the onset of wetness can be detected by a change of impedance by conductive strips **108a** and **108b.** The accuracy of the beginning of sufficient saturation of the colorimetric analyte sensing element **30** can be improved by sequentially activating each of the narrow beam LEDs and comparing the light detected by photodetector **124.** If there is a significant difference among the data returned by the photodetector **124** as a result of different narrow beam LEDs, the colorimetric analyte sensing element **30** may not be sufficiently saturated for reliable analysis. Therefore, in this embodiment, the system may begin collecting optical data relating to the colorimetric analyte sensing element **30** after a predetermined time period following bodily fluid contact with the colorimetric analyte sensing element **30** as determined by (1) a change of impedance by conductive strips **108a** and **108b** and (2) relatively consistent data returned by the photodetector **124** as a result of different narrow beam LEDs indicating substantially uniform wetness of the colorimetric analyte sensing element **30.**

Although the embodiment described above is an embodiment of a system **10** for measuring analyte concentration in an absorbent article which has an indicator component **20** and a durable component **100,** it is envisioned that in some cases durable component **100** can be combined with a plurality of indicator components **20** to create a kit for measuring analyte concentration in an absorbent article. The kit has at least one, preferably one or more, individually packaged indicator components **20.** This allows for the kit to measure analyte concentration in an absorbent article daily, or weekly, or monthly, or one or more times a day, or week or month. When used in this manner, system **10** is used to track changes in measured analyte concentration over the course of days, week, months, or even years.

Disposable absorbent articles for use in system **10** for measuring analyte concentrations include absorbent hygiene articles such as diapers (including infant diapers, training pants and adult incontinence products) and pads (including feminine sanitary napkins and pantiliners and nursing pads).

For example, an absorbent article for use in system **10** for measuring analyte concentrations is a diaper, and analyte concentrations are being measured in urine. Indicator component **20** has attachment means such as an adhesive layer **90.** Adhesive layer **90** is used to attach, or couple, indicator component **20** of system **10** to the fluid transport layer of the diaper. System **10** may be attached to a body-facing surface of the diaper. Other attachment means will be readily apparent, including without limitation, mechanical fasteners, such as clips, clamps, hook-and-loop systems, and bands; magnetic (including static electricity); friction; and the like. Indicator component may be arranged and configured for releasable attachment to a diaper.

The system for measuring analyte concentration in an absorbent article may have a plurality of colorimetric analyte sensing elements. FIGs. 9 to 17 show a system for measuring analyte concentration in an absorbent article of the present invention. System **200** has an indicator component **220** and a durable component **300.** FIGs. 9 and 10 are top and bottom, respectively, of system **200** when full assembled.

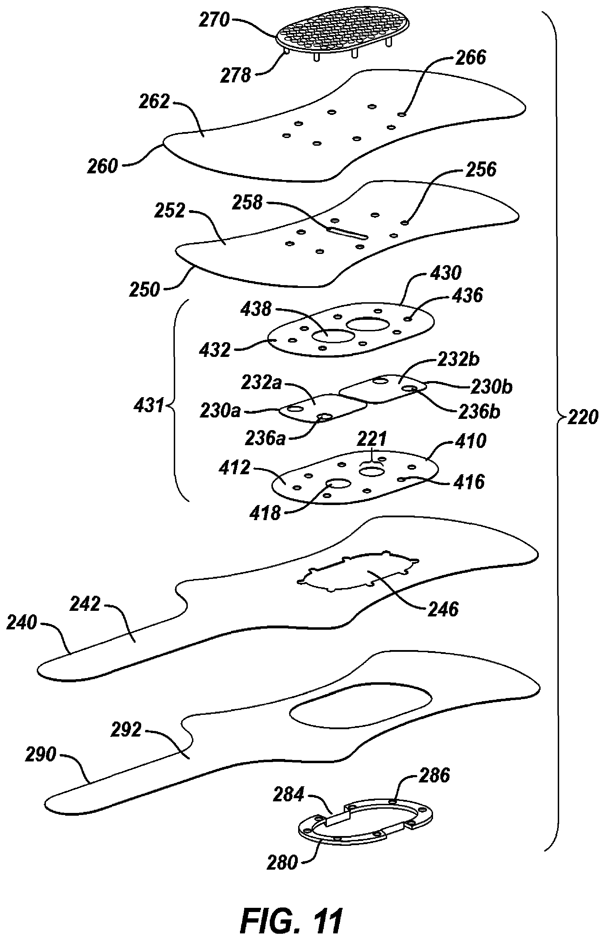

Indicator component **220** is shown in exploded view in FIG. 11. Indicator component **220** includes an indicator zone **221** that has a pair of colorimetric analyte sensing elements, first colorimetric analyte sensing element **230a,** and second colorimetric analyte sensing element **230b.** First colorimetric analyte sensing element **230a** has a first side **232a** and a second side **234a,** as well as perforations **236a.** Second colorimetric analyte sensing element **230b** has a first side **232b** and a second side **234b,** as well as perforations **236b.**

Colorimetric analyte sensing elements **230a, 230b** may be reagent impregnated matrices designed to produce a visual indication of the presence of a preselected analyte in sample produced by the wearer of system **200.** Chemistries and methods of detecting analytes by producing a visual indication are well known in the art. The preselected analyte measured by system **200** may be, glucose, ketones, bilirubin, blood, pH, protein, urobilinogen, nitrite, leukocytes, and/or creatinine, among others.

Colorimetric analyte sensing elements **230a, 230b** may be designed to produce a visual indication of the presence of the same preselected analyte in sample produced by the wearer of system **200.** In this case, colorimetric analyte sensing elements **230a, 230b** act to confirm the analysis. Colorimetric analyte sensing elements **230a, 230b** may also be designed to produce a visual indication of the presence of different preselected analytes in sample produced by the wearer of system **200.**

Again, the absorbent article may be a diaper, the fluid being tested is urine and the preselected analyte measured by system **200** is glucose. Glycosuria, or glucose in the urine, is the presence of higher than normal levels of sugar in the urine and may be due to complications with one's kidneys or diabetes.

The preselected analytes measured by system **200** may also be ketones. If cells in the body do not get sufficient glucose, the body burns fat for energy instead. This produces ketones which can show up in the blood and urine. High ketone levels in urine may indicate diabetic ketoacidosis (DKA), a complication that can lead to a coma or even death.

Some of the most common causes of glucose or ketones in the urine include: diabetes mellitus, hyperthyroidism, benign glycosuria, liver cirrhosis, or a high sugar diet. In addition, in some embodiments, one of ordinary skill in the art will recognize that choosing appropriate biosensor(s) capable of converting a preferred biomarker into a calorimetrically readable result could be used in genomics, transcriptomics, metabolomics and proteomics as well to determine the presence of inflammatory biomarkers that are present in urine.

The other components of indicator component **220** include an optional top plate **270,** an optional first flexible web **260,** a fluid transport layer **250,** a second flexible web **240,** an adhesive layer **290,** and a coupler shown here as holding plate **280.**

Colorimetric analyte sensing elements **230a, 230b** are encapsulated between first encapsulation layer **410** and second encapsulation layer **430** to form a fluid impervious envelope **431.** First encapsulation layer **410** has a first side **412** and a second side **414,** as well as perforations **416** and apertures **418.** Second encapsulation layer **430** has a first side **432** and a second side **434,** as well as perforations **436** and apertures **438.**

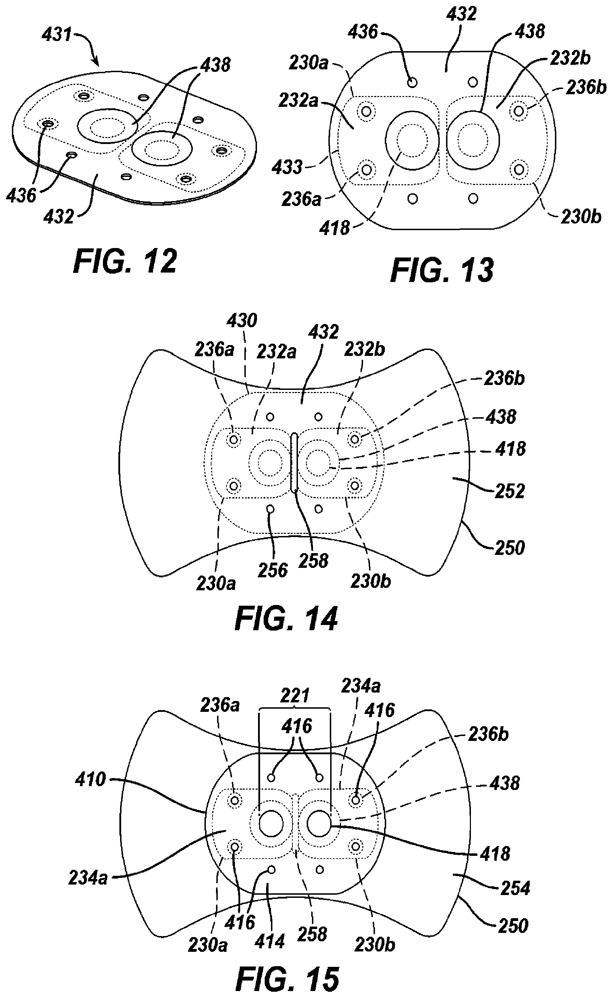

FIG. 12 is a top perspective view of the fluid impervious envelope **431** encapsulating colorimetric analyte sensing elements **230a, 230b** of indicator component **220** of system **200.** FIG. 13 shows a top view of the fluid impervious envelope **431** encapsulating colorimetric analyte sensing elements of FIG. 12. The figures show, in solid lines, first side **432,** perforations **436** and aperture **438** of second encapsulation layer **430.** In dashed lines, the figures show colorimetric analyte sensing elements **230a, 230b,** their first sides **232a, 232b** and perforations **236a, 236b** as well as apertures **418** of first encapsulation layer **410.** The dashed lines showing colorimetric analyte sensing elements **230a, 230b,** also outline discrete pockets **433** (one of two shown in FIG. 13) formed when first encapsulation layer **410** and second encapsulation layer **430** are sealed together where their surfaces contact.

When assembled, first perforations **436** of second encapsulation layer **430** are in alignment with perforations **236a, 236b** of colorimetric analyte sensing elements **230a, 230b,** as well as perforations **416** of first encapsulation layer **410** (not shown). In addition, apertures **438** of second encapsulation layer **430** are in alignment with apertures **418** of first encapsulation layer **410.**

The fluid impervious envelope **431** encapsulating colorimetric analyte sensing elements **230a, 230b** of indicator component **220** of system **200** is disposed on fluid transport layer **250.** This partially assembled indicator component of the system **200** is shown in a top in view FIG. 14, and in bottom view in FIG. 15. FIG. 14 shows, in solid lines, first side **252,** first perforations **256** and second perforation **258** of fluid transport layer **250.** In dashed lines, the figures show colorimetric analyte sensing elements **230a, 230b,** their first sides **232a, 232b** and perforations **236a, 236b,** as well as apertures **418** of first encapsulation layer **410** and first side **432** and apertures **438** of second encapsulation layer **430.**

FIG. 15 shows, in solid lines, second side **252** of fluid transport layer **250,** as well as second side **414,** perforations **416** and apertures **418** of first encapsulation layer **410.** In dashed lines, the figures show colorimetric analyte sensing elements **230a, 230b,** their second sides **234a, 234b** and perforations **236a, 236b,** and second perforation **258** of fluid transport layer **250.**

Second flexible web **240** has a first side **242,** a second side **244** and opening **246,** and is made of non-absorbing material, such as a polyethylene foam. The fluid impervious envelope **431** encapsulating colorimetric analyte sensing elements **230a, 230b** is disposed on second flexible web **240,** specifically in opening **246** of second flexible web **240** and is in fluid communication with fluid transport layer **250.** Fluid transport layer **250,** in turn, is in fluid communication with first flexible web **260.** First flexible web **260** has a first side **262,** and perforations **266,** and is made of a non-absorbing apertured film, such as a polyethylene mesh.

Second flexible web **240,** fluid transport layer **250,** and first flexible web **260** are designed to control the transport of bodily fluids to the colorimetric analyte sensing elements **230a, 230b** and to limit cross-contamination of fluids among different colorimetric analyte sensing elements. In use, fluid from the absorbable article first contacts first side **262** of first flexible web **260.** Since first flexible web **260** is a non-absorbing apertured film, fluid passes through first flexible web **260** and contacts first side **252** of fluid transport layer **250.** The fluid then permeates throughout fluid transport layer **250.** The fluid will contact first side **242** of second flexible web **240.** But, since second flexible web **240** is made of non-absorbing material, fluid in transport layer **250** does not penetrate second flexible web **240.** Finally, the fluid in transport layer **250** passes through apertures **438** of second encapsulation layer **430** to contact the colorimetric analyte sensing elements **230a, 230b.** Cross-contamination between the two colorimetric analyte sensing elements is eliminated or at least made insignificant and not detectable by means of the fluid barrier defined by the gap in capillarity within the fluid transport layer **250** provided by the second perforation **258.**

Sensing elements **230a, 230b,** first encapsulation layer **410,** second encapsulation layer **430,** second flexible web **240,** fluid transport layer **250,** and first flexible web **260** are stacked, as shown in FIG. 11, and are held together by top plate **270** and holding plate **280.** Top plate **270** has pins **278.** Pins **278** sequentially pass through perforations **266** of first flexible web **260,** perforations **256** of fluid transport layer **250,** perforations **416** of first encapsulation layer **410,** perforations **236a, 236b** of colorimetric analyte sensing elements **230a, 230b,** first perforations **436** of second encapsulation layer **430,** opening **246** of second flexible web **240,** and are finally disposed in blind holes **286** of holding plate **280.** A friction fit between top plate pins **278** and blind holes **286** hold the components of indicator component **220** together. Alternative assemblies may be held together by other interactions, such as snap fit, ultrasonic weld, heat weld, other mechanical fasteners, and the like.

Top plate **270** may have one or more channels on the side facing first side **262** of first flexible web **260.** The channel(s) may help direct fluid from the absorbent article to the first side **262** of first flexible web **260.**

Indicator component **220** may have attachment means, such as adhesive layer **290.** Adhesive layer **290** has a first side **292,** and is used to attach, or couple, indicator component **220** of system **200** to the fluid transport layer of the absorbent article, such as a diaper.

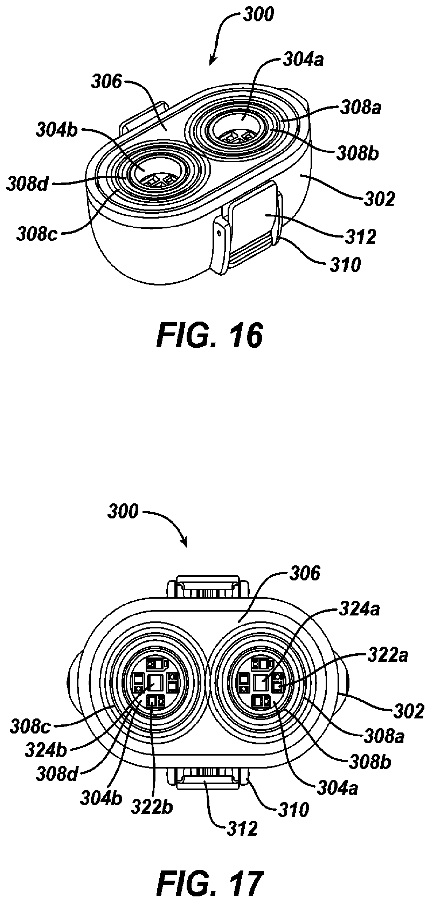

A durable component **300** of the system is shown in top perspective view in FIG. 16, and in top view in FIG. 17. Durable component **300** has a housing **302** with a pair of windows, first window **304a,** and second window **304b.** Durable component **300** also has a flat top surface **306.** A pair of spectrophotometers are disposed in housing **302.** The first spectrophotometer is adjacent to and in optical communication with first window **304a.** The components of the first spectrophotometer include light sources **322a** and photodetector **324a.** First spectrophotometer is in optical communication with colorimetric analyte sensing element **230a.** The second spectrophotometer is adjacent to and in optical communication with second window **304b.** The components of the second spectrophotometer include light sources **322b** and photodetector **324b.** Second spectrophotometer is in optical communication with colorimetric analyte sensing element **230b.** While the durable component **300** has been shown with two spectrophotometers, additional spectrophotometers may be included for measurements of additional analytes or bodily fluid conditions, such as pH, temperature, etc. Indicator zone **221** is the area of indicator component **220** where colorimetric analyte sensing element **230a** is exposed to light sources **322a.**

Thought not shown, durable component **300** also has a printed circuit board (PCB) with a computing system having one or more processors and a memory, as well as means for electronic communication to send the results of analyses to data processing systems that are external to system **200.** Data processing systems that may be used include at least one external device including server computers, client computers, and handheld devices such as cellphones.

As shown in FIGs. 16 and 17, the first and second spectrophotometer may include four light sources **322a, 322b** and each spectrophotometer has one photodetector **324a, 324b.** Each spectrophotometer may have associated therewith at least one light sources **322a, 322b.** Each spectrophotometer may include at least six or more light sources **322a, 322b.** As mentioned earlier, light sources **322a, 322b** may be light-emitting diodes (LEDs), and more specifically, RGB LEDs. Light sources **322a, 322b** may sequentially emit red light, green light, and blue light, allowing for the near simultaneous collection of three data points, or, the sequence of emitted red light, green light, and blue light may vary.

The photodetectors in the spectrometers **324a, 324b,** as discussed previously, may specifically measure the red, green, and blue light, allowing data to be obtained in narrow wavelength regions. The light waves emitted from light sources **322a** reflect off of colorimetric analyte sensing element **230a,** and the reflected light is measure by photodetector **324a.** The light waves emitted from light sources **322b** reflect off of colorimetric analyte sensing element **230b,** and the reflected light is measure by photodetector **324b.** The components of the spectrophotometer may be coated with a protective material. The protective material keeps the moisture from the colorimetric analyte sensing elements **230a, 230b** from contacting, and potentially damaging, the components of the spectrophotometers.

FIGs. 16 and 17 also show connectors **310** disposed on housing **302.** Connectors **310** comprise a standard spring-loaded clips **312** that are biased to hold clips **312** to housing **302** of durable component **300.** As shown in FIG. 11, holding plate **280** has receiving elements **286** disposed thereon. To releasably attach durable component **300** to holding plate **280,** clips **312** are fastened to receiving elements **286.** By this means, durable component **300** is releasably attach to indicator component **220.** Other attachment means will be readily apparent, including without limitation, mechanical fasteners, such as clamps, hook-and-loop systems, threaded apertures, bayonet couplings, straps, belts, and bands; magnetic (including static electricity); friction; and the like.

FIG. 17 also shows conductive strips **308a, 308b, 308c** and **308d** disposed on top surface **306** of durable component **300.** Conductive strips **308a, 308b, 308c** and **308d** act as moisture sensors, arranged and configured to communicate the presence of moisture in colorimetric analyte sensing elements **230a, 230b** to the computing system disposed in durable component **300.** As shown in FIG. 17, conductive strips **308a** and **308b** are associated with first window **304a** and colorimetric analyte sensing element **230a.** Conductive strips **308c** and **308d** are associated with second window **304b** and colorimetric analyte sensing element **230b.** The computing system disposed in durable component **300** is operatively connected to the moisture sensors as well as the components of the spectrophotometer.

Conductive strips **308a** and **308b** are adjacent to colorimetric analyte sensing element **230a.** When moisture impinges on colorimetric analyte sensing element **230a,** it will also contact portions of conductive strips **308a** and **308b.** Conductive strips **308c** and **308d** are adjacent to colorimetric analyte sensing element **230b.** When moisture impinges on colorimetric analyte sensing element **230b,** it will also contact portions of conductive strips **308c** and **308d.**

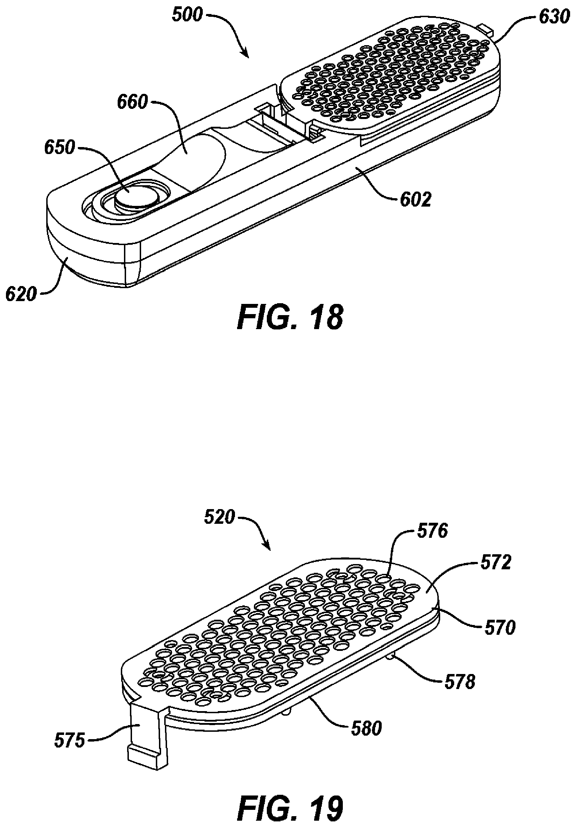

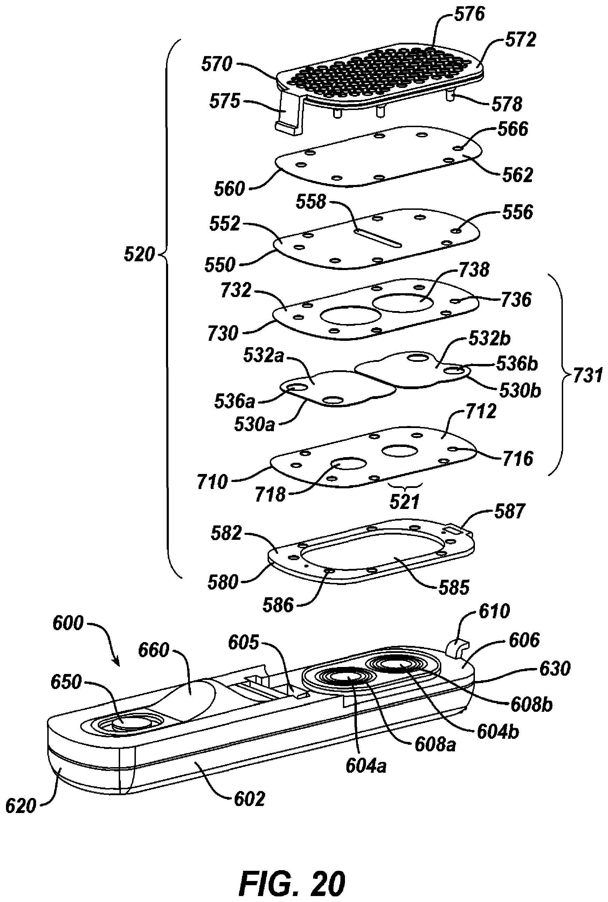

The mode of operation of conductive strips **308a, 308b, 308c** and **308d** as moisture sensors are identical to the operation of conductive strips **108a** and **108b** as described in FIGs. 7 and 8. The moisture front progresses partially, and finally, fully across conductive strips **308a** and **308b,** and **308c** and **308d.** The system for measuring analyte concentrations in bodily fluids may be used in an absorbent article, or it may be directly contacted by bodily fluids outside of an absorbent article. For example, the system can contact bodily fluids collected in a specimen container or may come into contact with bodily fluids such as urine as the fluid is expelled from the human body. FIGs. 18 to 20 show a system for measuring analyte concentration in a bodily fluid of the present invention. System **500** has an indicator component **520** and a durable component **600.** FIG. 18 is a top perspective view of system **500** when full assembled. FIG. 19 is a top perspective view of the indicator component **520** of system **500.** FIG. 20 is a partially exploded view of system **500,** where indicator component **520** is shown in exploded view.

In FIG. 20, indicator component **520** includes an indicator zone **521** is shown to have a pair of colorimetric analyte sensing elements, first colorimetric analyte sensing element **530a,** and second colorimetric analyte sensing element **530b.** First colorimetric analyte sensing element **530a** has a first side **532a** and perforations **536a.** Second colorimetric analyte sensing element **530b** has a first side **532b** and perforations **536b.**

As discussed before, colorimetric analyte sensing elements **530a, 530b** may be reagent impregnated matrices designed to produce a visual indication of the presence of a preselected analyte in sample produced by the user of system **500.** The preselected analyte measured by system **500** may be, glucose, ketones, bilirubin, blood, pH, protein, urobilinogen, nitrite, leukocytes, and/or creatinine, among others.

Again, colorimetric analyte sensing elements **530a, 530b** may be designed to indicate the presence of the same preselected analyte in sample produced by the user of system **500.** In this case, colorimetric analyte sensing elements **530a, 530b** act to confirm the analysis. Colorimetric analyte sensing elements **530a, 530b** may also be designed to produce a visual indication of the presence of different preselected analytes in sample produced by the user of system **500.**

Again, the fluid being tested may be urine and the preselected analyte measured by system **500** is glucose, one or more ketones, or combinations thereof. The presence of higher than normal levels of glucose and/or ketones in the urine and may be due to complications with the user's kidneys, or other conditions such as diabetes mellitus, hyperthyroidism, benign glycosuria, liver cirrhosis, or a high sugar diet.

In addition, choosing appropriate biosensor(s) capable of converting a preferred biomarker into a calorimetrically readable result may be used in genomics, transcriptomics, metabolomics, and proteomics as well to determine the presence of inflammatory biomarkers that are present in urine or other bodily fluids.

The other components of indicator component **520** include a top plate **570,** a first flexible web **560,** a fluid transport layer **550,** a first encapsulation layer **710,** a second encapsulation layer **730,** and a coupler shown here as holding plate **580.**

Colorimetric analyte sensing elements **530a, 530b** are encapsulated between first encapsulation layer **710** and second encapsulation layer **730** to form a fluid impervious envelope **731.** First encapsulation layer **710** has a first side **712,** perforations **716,** and apertures **718.** Second encapsulation layer **730** has a first side **732,** perforations **736,** and apertures **738.**

When assembled in indicator component **520,** perforations **716** of first encapsulation layer **710** are in alignment with perforations **536a, 536b** of colorimetric analyte sensing elements **530a, 530b,** as well as perforations **736** of second encapsulation layer **730.** In addition, apertures **718** of first encapsulation layer **710** are in alignment with apertures **738** of second encapsulation layer **730.**

FIG. 20 also shows fluid transport layer **550** and first flexible web **560.** When assembled in indicator component **520,** fluid transport layer **550** is disposed on encapsulated colorimetric analyte sensing elements **530a, 530b** of indicator component **520** of system **500.** Fluid transport layer **550** has first side **552,** first perforations **556** and second perforation **558.** First flexible web **560** is disposed on fluid transport layer **550,** and has a first side **562,** and perforations **566,** and is made of a non-absorbing apertured film, such as a polyethylene mesh.

When assembled in indicator component **520,** colorimetric analyte sensing elements **230a, 230b,** which are encapsulated in the fluid impervious envelope **731,** are in fluid communication with fluid transport layer **550.** Fluid transport layer **550,** in turn, is in fluid communication with first flexible web **560.**

Fluid transport layer **550** and first flexible web **560** are designed to control the transport of bodily fluids to the colorimetric analyte sensing elements **530a, 530b** and to limit cross-contamination of fluids among different colorimetric analyte sensing elements. In use, bodily fluid first contacts first side **562** of first flexible web **560.** Since first flexible web **560** is a non-absorbing apertured film, fluid passes through first flexible web **560** and contacts first side **552** of fluid transport layer **550.** The fluid then permeates throughout fluid transport layer **550.** Finally, the fluid in transport layer **550** passes through apertures **738** of second encapsulation layer **730** to contact the colorimetric analyte sensing elements **530a, 530b.** Again, cross-contamination between the two colorimetric analyte sensing elements is eliminated or at least made insignificant and not detectable by means of the fluid barrier defined by the gap in capillarity within the fluid transport layer **550** provided by the second perforation **558.**

Sensing elements **530a, 530b,** first encapsulation layer **710,** second encapsulation layer **730,** fluid transport layer **550,** and first flexible web **560** are stacked, as shown in FIG. 20, and are held together by top plate **570** and holding plate **580.** Top plate **570** has pins **578.** Pins **578** sequentially pass through perforations **566** of first flexible web **560,** perforations **556** of fluid transport layer **550,** perforations **716** of first encapsulation layer **710,** perforations **536a, 536b** of colorimetric analyte sensing elements **530a, 530b,** perforations **736** of second encapsulation layer **730,** and are finally disposed in blind holes **586** on first side **582** of holding plate **580.** A friction fit between top plate pins **578** and blind holes **586** hold the components of indicator component **520** together. Alternative assemblies may be held together by other interactions, such as snap fit, ultrasonic weld, heat weld, other mechanical fasteners, and the like.

Top plate **570** has apertures **576** which help direct fluid to first side **562** of first flexible web **560.** Top plate **570** also has disposed thereon protrusion **575.** Protrusion **575,** as well as protrusion **587** disposed on holding plate **580** are means of attaching indicator component **520** to durable component **600** of system **500.**

Durable component **600** is shown in top perspective view in FIG. 20. Durable component **600,** with proximal end **620** and distal end **630,** has a housing **602** with a pair of windows, first window **604a,** and second window **604b.** Durable component **600** also has a flat top surface **606,** conductive rings **608a** and **604b,** receiving element **605,** protrusion **610,** activation button **650,** and finger grip **660.**

Though not shown, a pair of spectrophotometers are disposed in housing **602.** The first spectrophotometer is adjacent to and in optical communication with first window **604a,** while the second spectrophotometer is adjacent to and in optical communication with second window **604b.** The first spectrophotometer is in optical communication with colorimetric analyte sensing element **530a,** and the second spectrophotometer is in optical communication with colorimetric analyte sensing element **530b.** While the durable component **600** has been shown with two spectrophotometers, additional spectrophotometers may be included for measurements of additional analytes or bodily fluid conditions, such as pH, temperature, etc. Indicator zone **521** is the area of indicator component **520** where colorimetric analyte sensing element **530a** is exposed to light source(s).

Thought not shown, durable component **600** also has a printed circuit board (PCB) with a computing system having one or more processors and a memory, as well as means for electronic communication to send the results of analyses to data processing systems that are external to system **500.** Data processing systems that may be used include at least one external device including server computers, client computers, and handheld devices such as cellphones.

As discussed in the other embodiments of this document, the spectrophotometers may include at least one or more, or two or more, or four or more, or six or more light sources and at least one, or at least two or more photodetectors. Also, as mentioned earlier, light sources in durable component **600** may be light-emitting diodes (LEDs), and more specifically, RGB LEDs. The light sources may sequentially emit red light, green light, and blue light, allowing for the near simultaneous collection of three data points, or, the sequence of emitted red light, green light, and blue light may vary.

Photodetectors in durable component **600** also, as discussed previously, may specifically measure the red, green, and blue light, allowing data to be obtained in narrow wavelength regions, and may be coated with a protective material to reduce the possibility of damage to their components.

FIG. 18 shows a top perspective view of durable component **600** and indicator component **520** assembled to form system **500.** Here, indicator component **520** is disposed on distal end **630** of durable component **600.** Top plate **570** of durable component **600** has protrusion 575, and holding plate **580** has protrusion **587.** Durable component **600** has receiving element **605** and protrusion **610.** To releasably attach indicator component **520** to durable component **500,** protrusion **575** of top plate **570** is disposed in receiving element **605** of durable component **600.** Then, protrusion **587** of holding plate **580** is engaged with protrusion **610** of durable component **600** with a snap connection.

FIG. 20 shows conductive strips **608a** and **608b** disposed on top surface **606** of durable component **600.** Conductive strips **608a** and **608b** act as a moisture sensor in system **500.** They are arranged and configured to communicate the presence of moisture in colorimetric analyte sensing elements **530a, 530b** to the computing system disposed in durable component **600.** In this embodiment, conductive strips **608a** are associated with first window **604a** and colorimetric analyte sensing element **530a.** Conductive strips **308b** are associated with second window **604b** and colorimetric analyte sensing element **530b.** The computing system disposed in durable component **600** is operatively connected to the moisture sensors as well as the components of the spectrophotometer.

The mode of operation of conductive strips **608a** and **608b** as moisture sensors are identical to the operation of conductive strips **108a** and **108b** as described in FIGs. 7 and 8. The moisture front progresses partially, and finally, fully across conductive strips **608a** and **608b.**

A durable component may be matched with a plurality of indicator components to create a kit for measuring analyte concentration in an absorbent article comprising. For example, the kit may have a durable component **100, 300, 600** (described above) and a plurality of indicator components, **20, 220, 520** (also described above). To ensure integrity of the indicator components during storage, each such indicator component is enclosed in an individual package.

The present disclosure also includes a method of measuring analyte concentration in an absorbent article. Bodily fluid is collected and transported via a transport layer to at least one colorimetric analyte sensing element. The presence of the bodily fluid at the at least one colorimetric analyte sensing element begins a countdown for a predetermined time period. Optical data relating to the colorimetric analyte sensing element is collected by at least one spectrophotometer after the predetermined time period. The optical data is communicated to a computing system having at least one processor and data storage. The optical data is analyzed to determine at least one analyte concentration in the bodily fluid.

The predetermined time period following bodily fluid contact with the colorimetric analyte sensing element could be greater than 15 seconds, or greater than 30 seconds, or greater than 60 seconds, or greater than 120 seconds, or greater than 240 seconds, or greater than 300 seconds, or greater than 360 seconds or more. The predetermined time period following bodily fluid contact with the colorimetric analyte sensing element could be a predetermined time range, for example, from about 15 to about 360 seconds, or from about 30 to about 240 seconds, or from about 120 to about 180 seconds, or from about 240 to about 360 seconds.

The analyte measured by system may be, glucose, ketone, bilirubin, blood, pH, protein, urobilinogen, nitrite, leukocytes, and/or creatinine, among others.

The analytes found in bodily fluids potentially carries evidence of developing metabolic system problems. There is a desire for people in and out of the medical establishment to track and analyze changes in the concentration of analytes in bodily fluids over time. These changes may be useful for predicting risk of a future disease conditions. Therefore, the systems discussed in the present invention allow for a method for predicting risk of a future disease condition.

As above, bodily fluid is collected and transported via a transport layer to at least one colorimetric analyte sensing element. The presence of the bodily fluid at the at least one colorimetric analyte sensing element begins a countdown for a predetermined time period. Optical data relating to the colorimetric analyte sensing element is collected by at least one spectrophotometer after the predetermined time period. The optical data is communicated to a computing system having at least one processor and data storage. The optical data is analyzed to determine at least one analyte concentration in the bodily fluid. A threshold analyte concentration of the at least one analyte concentration that indicates the risk of developing a future disease condition is compared against the at least one analyte concentration, and this can be recorded over time. Thus, the risk of developing a future disease condition may be monitored over time.

The system may be arranged, configured, and programmed with multiple photodetectors 124 and multiple colorimetric analyte sensing elements 30 to determine multiple analyte concentrations in the bodily fluid.

Non-invasively measuring the analyte concentration in bodily fluids is also ideally suited for epidemiological studies to rapidly identify problems prevalent in specific geographies or for specific populations of people. The analyte concentration measurements from system **10** may be collected over a wide population for long periods of time. The collected data may be studied to determine relationship between various analyte levels and disease states, or combined with other physiological parameter such as blood pressure, blood oxygen level, and pulse rate, or with vital statistics such as age, sex, weight, and nationality, to create a predictive model of future disease states as a function of the saved parameters.

The foregoing methods may employ a system deployed in or in conjunction with an absorbent article, such as a diaper or pad, or they may employ directly contacting bodily fluids without the use of an absorbent article. For example, system **500** may be attached to a body-facing surface of the diaper. System **500** of FIGs. 18-20 may be directly contacted with bodily fluids. It may be dipped into bodily fluids that are first collected in a specimen container by grasping system **500** by finger grip **660** on proximal end **620** of durable component **600.** System **500** may be energized by user engaging activation button **650** on proximal end **620** of durable component **600** before or after placing distal end **630** into specimen container. Alternatively, the indicator component of system **500** may be placed in a stream of bodily fluids such as urine as the fluid is expelled from the human body. In these uses, durable component **600** is a handheld analyzer.

### EXAMPLES

Example 1: Demonstration of the stability of reflectance values versus time in a colorimetric analyte sensing element.

To test the changes in reflectance values versus time, measurements of reflective values were performed with a prototype spectrophotometer on a series of prototype colorimetric analyte sensing elements which were exposed to glucose solutions at room temperature.

The prototype spectrophotometer was constructed using the following elements:
- Light sources **122:** RGB LEDs with light wavelengths 624, 525, and 468 nm from INOLUX (Santa Clara, CA). The part number is IN-S66TATRGB.
- Photodetector **124:** An integrated circuit (IC) color light-to-digital converters with Infrared (IR) Filter. The integrated circuit provides digital values of Red, Green, Blue (RGB), and clear light sensing. An IR blocking filter minimized the IR light spectral component which allowing color measurements to be made accurately. The part number was TCS34725, available from ams AG (Premstaetten, Austria).

The prototype colorimetric analyte sensing elements **30** were porous polysulfone membranes from PortaScience Inc. (Moorsetown, NJ). The membranes were infused with the following mixture:
- Glucose oxidase: 16.3% W/W
- Horseradish peroxidase: 0.6% W/W
- Potassium iodide: 7% W/W
- 60.7% W/W buffer, and
- 16.7% W/W non-reactive ingredients.

The tests were performed with artificial urine solutions with a glucose concentration of 25 milligrams/deciliter. All tests were performed at room temperature.

Light scans at three wavelengths (red, green, blue) were performed on dry colorimetric analyte sensing elements so that a baseline color of the element was established. Then, the colorimetric analyte sensing elements were saturated with the artificial urine solutions. Measurements of reflectivity were performed at three channels (red, green, and blue) of light every thirty seconds, and reflectivity was recorded.

Table 1.1 shows the reflectivity off of the saturated colorimetric analyte sensing elements at each wavelength at each time point.

**Table 1.1: Reflectivity measurements in three light channels of colorimetric analyte sensing elements saturated in artificial urine solutions containing a 25 milligrams/deciliter glucose.**

| **Time (seconds)** | **Red** | **Green** | **Blue** |
|---|---|---|---|
| 0 | 1478 | 2422 | 3882 |
| 30 | 1250 | 1885 | 3668 |
| 60 | 1169 | 1736 | 3600 |
| 90 | 1127 | 1663 | 3569 |
| 120 | 1096 | 1613 | 3541 |
| 150 | 1075 | 1575 | 3518 |
| 180 | 1056 | 1546 | 3499 |

The table shows reflectivity of light at each of the tested wavelengths decreased as time increased.

Next, relative variation of the traces were calculated using the following equation: $Relative Variation \left({t}_{2}\right) = 100 * \left[Reflectance \left({t}_{1}\right)-Reflectance \left({t}_{2}\right)\right] / Reflectance \left({t}_{0}\right) ,$ where:
Reflectance (t₁) and Reflectance (t₂) are the reflectance measurements at time 1 and time 2, respectively, and
Reflectance (t₀) is the reflective measurement of the dry colorimetric analyte sensing element.

The units of the Relative Variation are percent (%).

For example, using the reflective measurements for the green channel from Table 1.1, the Relative Variation was at 60 seconds was calculated as: $Relative Variation \left({t}_{60}\right) = 100 * \left[1885-1736\right] / 2422 = 6.15 %$

Table 1.2 shows the Relative Variation of the reflective measurements for the green channel from the colorimetric analyte sensing elements at each time point.

**Table 1.2: Relative Variation of the green channel reflective measurements versus time.**

| **Time (seconds)** | **Relative variation - Green channel** |
|---|---|
| 0 | Reference |
| 30 | 22.17% |
| 60 | 6.15% |
| 90 | 3.01% |
| 120 | 2.06% |
| 150 | 1.57% |
| 180 | 1.20% |

The table shows, the last three values of Relative Variation converging. So, in this example, the data taken 150 seconds after the colorimetric analyte sensing element is saturated with glucose solution may be used in the algorithm to assess the glucose concentration. Or the algorithm may use the data taken between 120 and 180 seconds after the colorimetric analyte sensing element is saturated with glucose solution in testing for glucose.

In other embodiments, the converging values of Relative Variation may be used to dictate the appropriate time to record data. So, for example, when Relative Variation falls below two percent, or one and a half percent, the algorithm may choose that point in time as the time to record the data.

Of course, the limits of this test when compared to realistic conditions include the temperature of the solution as well as the rate real urine will saturate the colorimetric analyte sensing element in the system. However, this qualitative example could be reflective of the real process. Extensive testing in realistic conditions must be continued.

Example 2: Demonstration of the stability of reflectance values versus time in a colorimetric analyte sensing element with a moisture sensor.

As mentioned above, conductive strips **108a** and **108b** act as a moisture sensor in system **10** and are arranged and configured to communicate the presence of moisture in colorimetric analyte sensing element **30** to the computing system disposed in durable component **100.** In this example, measurements of reflective values were performed with a prototype spectrophotometer on a series of prototype colorimetric analyte sensing elements which were exposed to glucose solutions at room temperature, where a moisture sensor was used to initiate the testing for analytes in the colorimetric sensor.

The prototype spectrophotometer and prototype colorimetric analyte sensing elements were the same as those used in Example 1, as was the artificial urine solution (glucose concentration of 25 milligrams/deciliter). As in Example 1, all tests were performed at room temperature.

The test was performed as follows:
1. 1.5 ml of the artificial urine was applied at the periphery of the prototype colorimetric analyte sensing element at the 3 o'clock position.
2. The capacitor moisture sensor showed full wetting of the sensor in less than 20 seconds.
3. The prototype colorimetric analyte sensing element was placed upside on prototype spectrophotometer and measurements of reflectivity were performed at three channels (red, green, and blue) of light every sixty seconds. The reflectivity was recorded.

Table 2.1 shows the reflectivity off of the saturated colorimetric analyte sensing elements at each wavelength at each time point.

**Table 2.1: Reflectivity measurements in three light channels of colorimetric analyte sensing elements saturated in artificial urine solutions containing a 25 milligrams/deciliter glucose.**

| **Time (seconds)** | **Red** | **Green** | **Blue** |
|---|---|---|---|
| 0 | 2382 | 3822 | 6877 |
| 60 | 2057 | 3215 | 6739 |
| 120 | 1953 | 3053 | 6658 |
| 180 | 1890 | 2957 | 6605 |
| 240 | 1863 | 2892 | 6587 |
| 300 | 1838 | 2850 | 6556 |
| 360 | 1821 | 2823 | 6530 |

The Relative Variation of the reflective measurements for the green channel were calculated as shown in Example 1. Table 2.2 shows the Relative Variation of the reflective measurements at each time point.

**Table 2.2: Relative Variation of the green channel reflective measurements versus time.**

| **Time (seconds)** | **Relative variation - Green channel** |
|---|---|
| 0 | Reference |
| 60 | 15.88% |
| 120 | 4.24% |
| 180 | 2.51% |
| 240 | 1.70% |
| 300 | 1.10% |
| 360 | 0.71% |

The table shows, the last three values of Relative Variation show conversion of the data. So, in this example, the data taken 240 or 300 seconds after the colorimetric analyte sensing element is saturated with glucose solution may be good to use in the algorithm to assess the glucose concentration. Or the algorithm may use the data taken between 240 and 360 seconds after the colorimetric analyte sensing element is saturated with glucose solution in testing for glucose.

The specification, embodiments, and examples above are presented to aid in the complete and non-limiting understanding of the invention disclosed herein. Since many variations and embodiments of the invention can be made without departing from its scope, the invention resides in the claims hereinafter appended.

## Claims

1. A disposable indicator component (220, 520) for use in a system for measuring analyte concentration in a bodily fluid or for use with a handheld analyzer, comprising:
a) a first flexible web layer (260, 560);
b) a fluid transport layer (250, 550) adjacent the first flexible web layer (260, 560); and
c) a fluid impervious envelope (431, 731) surrounding an indicator zone (221, 521) adjacent the fluid transport layer (250, 550);
wherein:
I) the first flexible web layer (260, 560), fluid transport layer (250, 550), and the fluid impervious envelope (431, 731) are stacked in order and secured together;
II) the indicator zone (221, 521) comprises at least two colorimetric analyte sensing elements (230a, 230b, 530a, 530b);
III) the fluid impervious envelope (431, 731) has a discrete pocket arranged and configured to contain each of the at least two colorimetric analyte sensing elements (230a, 230b, 530a, 530b) and each pocket has a unique aperture in fluid communication with the fluid transport layer (250, 550);
IV) the fluid transport layer (250, 550) is arranged and configured to inhibit fluid transport between apertures in the fluid impervious envelope (431, 731).

2. A disposable indicator component (520) according to claim 1, wherein the disposable indicator component (520) is for use with a handheld analyzer.

3. The disposable indicator component (520) of claim 2 further comprising:
d) a top plate (570) adjacent the first flexible web layer; and
e) a holding plate (580),
wherein the first flexible web layer (560), fluid transport layer (550) and fluid impervious envelope (731) are secured between the top plate (570) and the holding plate (580), and the disposable indicator component (520) further comprises a coupler (580) for releasably attaching the disposable indicator component (520) to a housing for the handheld analyzer (600).

4. The disposable indicator component (520) of claim 3 wherein the top plate (570) and holding plate (580) are arranged and configured to provide a predetermined spacing to accommodate indicator component layers with predetermined fluid transport capacity to the indicator zone (521).

5. The disposable indicator component (520) of claim 2 enclosed within an individual package.

6. The disposable indicator component (220) of claim 1, wherein the disposable indicator component (220) is for use in a system for measuring analyte concentration in a bodily fluid, the disposable indicator component comprising:
a) a coupler (280) for coupling the indicator component to a component having at least one spectrophotometer (300) contained within a housing;
b) a top plate (270);
c) a second flexible web layer (240) adjacent the fluid impervious envelope;
d) attachment means disposed on the second flexible web layer (240); and
e) a holding plate (280);
wherein:
I) the first flexible web layer (260), fluid transport layer (250), fluid impervious envelope (431), and the second flexible web layer (240) are stacked in order and secured between the top plate (270) and the holding plate (280);
and wherein the fluid transport layer is in fluid communication with the indicator zone.

7. The disposable indicator component (220) of claim 6 wherein the top plate (270) and holding plate (280) are arranged and configured to provide a predetermined spacing to accommodate indicator component layers with predetermined fluid transport capacity to the indicator zone (221).

8. The disposable indicator component (220) of claim 6 wherein the fluid transport layer (250) comprises a fluid barrier disposed between apertures in the fluid impervious envelope (431).

9. The disposable indicator component (220) of claim 6 comprising a diaper.

10. The disposable indicator component (220) of claim 6 arranged and configured for releasable attachment to a diaper.

11. The disposable indicator component (220) of claim 6 enclosed within an individual package.

## Patentansprüche

1. Einweg-Indikatorkomponente (220, 520) zur Verwendung in einem System zur Messung der Analytkonzentration in einer Körperflüssigkeit oder zur Verwendung mit einem handgehaltenen Analysator, umfassend:
a) eine erste flexible Gewebelage (260, 560);
b) eine Fluidtransportschicht (250, 550) angrenzend an die erste flexible Gewebelage (260, 560); und
c) eine fluidundurchlässige Hülle (431, 731), die eine Indikatorzone (221, 521) umgibt, angrenzend an die Fluidtransportschicht (250, 550);
wobei:
I) die erste flexible Gewebelage (260, 560), die Fluidtransportschicht (250, 550) und die fluidundurchlässige Hülle (431, 731) in der Reihenfolge gestapelt und aneinander befestigt sind;
II) die Indikatorzone (221, 521) mindestens zwei kolorimetrische Analyterfassungselemente (230a, 230b, 530a, 530b) umfasst;
III) die fluidundurchlässige Hülle (431, 731) eine diskrete Tasche aufweist, die so angeordnet und konfiguriert ist, dass sie jedes der mindestens zwei kolorimetrischen Analyterfassungselemente (230a, 230b, 530a, 530b) enthält, und jede Tasche eine einzigartige Öffnung in Fluidverbindung mit der Fluidtransportschicht (250, 550) aufweist;
IV) die Fluidtransportschicht (250, 550) angeordnet und konfiguriert ist, um einen Fluidtransport zwischen Öffnungen in der fluidundurchlässigen Hülle (431, 731) zu verhindern.

2. Einweg-Indikatorkomponente (520) nach Anspruch 1, wobei die Einweg-Indikatorkomponente (520) zur Verwendung mit einem handgehaltenen Analysator bestimmt ist.

3. Einweg-Indikatorkomponente (520) nach Anspruch 2, ferner umfassend:
d) eine obere Platte (570) angrenzend an die erste flexible Gewebelage; und
e) eine Halteplatte (580),
wobei die erste flexible Gewebelage (560), die Fluidtransportschicht (550) und die fluidundurchlässige Hülle (731) zwischen der oberen Platte (570) und der Halteplatte (580) befestigt sind und die Einweg-Indikatorkomponente (520) ferner einen Koppler (580) zum lösbaren Anbringen der Einweg-Indikatorkomponente (520) an einem Gehäuse für den handgehaltenen Analysator (600) umfasst.

4. Einweg-Indikatorkomponente (520) nach Anspruch 3, wobei die obere Platte (570) und die Halteplatte (580) so angeordnet und konfiguriert sind, dass sie einen vorbestimmten Abstand vorsehen, um Indikatorkomponenten-Lagen mit einer vorbestimmten Fluidtransportkapazität in die Indikatorzone (521) aufzunehmen.

5. Einweg-Indikatorkomponente (520) nach Anspruch 2, die in einer individuellen Verpackung eingeschlossen ist.

6. Einweg-Indikatorkomponente (220) nach Anspruch 1, wobei die Einweg-Indikatorkomponente (220) zur Verwendung in einem System zur Messung der Analytkonzentration in einer Körperflüssigkeit dient, wobei die Einweg-Indikatorkomponente Folgendes umfasst:
a) einen Koppler (280) zum Koppeln der Indikatorkomponente an eine Komponente, die mindestens ein Spektralphotometer (300) aufweist, das in einem Gehäuse enthalten ist;
b) eine obere Platte (270);
c) eine zweite flexible Gewebelage (240) angrenzend an die fluidundurchlässige Hülle;
d) Befestigungsmittel, die auf der zweiten flexiblen Gewebelage (240) angeordnet sind; und
e) eine Halteplatte (280);
wobei:
I) die erste flexible Gewebelage (260), die Fluidtransportschicht (250), die fluidundurchlässige Hülle (431) und die zweite flexible Gewebelage (240) in der Reihenfolge gestapelt und zwischen der oberen Platte (270) und der Halteplatte (280) befestigt sind;
und wobei die Fluidtransportschicht in Fluidverbindung mit der Indikatorzone steht.

7. Einweg-Indikatorkomponente (220) nach Anspruch 6, wobei die obere Platte (270) und die Halteplatte (280) so angeordnet und konfiguriert sind, dass sie einen vorbestimmten Abstand vorsehen, um Indikatorkomponenten-Lagen mit einer vorbestimmten Fluidtransportkapazität in die Indikatorzone (221) aufzunehmen.

8. Einweg-Indikatorkomponente (220) nach Anspruch 6, wobei die Fluidtransportschicht (250) eine Fluidbarriere umfasst, die zwischen Öffnungen in der fluidundurchlässigen Hülle (431) angeordnet ist.

9. Einweg-Indikatorkomponente (220) nach Anspruch 6, umfassend eine Windel.

10. Einweg-Indikatorkomponente (220) nach Anspruch 6, die zur lösbaren Anbringung an einer Windel angeordnet und konfiguriert ist.

11. Einweg-Indikatorkomponente (220) nach Anspruch 6, die in einer individuellen Verpackung eingeschlossen ist.

## Revendications

1. Composant indicateur jetable (220, 520) pour une utilisation dans un système de mesure d'une concentration d'analyte dans un fluide corporel ou pour une utilisation avec un analyseur portatif, comprenant :
a) une première couche de bande souple (260, 560) ;
b) une couche de transport de fluide (250, 550) adjacente à la première couche de bande souple (260, 560) ; et
c) une enveloppe imperméable aux fluides (431, 731) entourant une zone indicatrice (221, 521) adjacente à la couche de transport de fluide (250, 550) ;
dans lequel :
I) la première couche de bande souple (260, 560), la couche de transport de fluide (250, 550) et l'enveloppe imperméable aux fluides (431, 731) sont empilées dans cet ordre et fixées ensemble ;
II) la zone indicatrice (221, 521) comprend au moins deux éléments de détection colorimétrique d'analyte (230a, 230b, 530a, 530b) ;
III) l'enveloppe imperméable aux fluides (431, 731) comporte une poche discrète agencée et conçue pour contenir chacun des au moins deux éléments de détection colorimétrique d'analyte (230a, 230b, 530a, 530b) et chaque poche comporte une ouverture unique en communication fluidique avec la couche de transport de fluide (250, 550) ;
IV) la couche de transport de fluide (250, 550) est disposée et conçue pour empêcher le transport de fluide entre des ouvertures dans l'enveloppe imperméable aux fluides (431, 731).

2. Composant indicateur jetable (520) selon la revendication 1, dans lequel le composant indicateur jetable (520) est destiné à être utilisé avec un analyseur portatif.

3. Composant indicateur jetable (520) selon la revendication 2, comprenant en outre :
d) une plaque supérieure (570) adjacente à la première couche de bande souple ; et
e) une plaque de maintien (580),
dans lequel la première couche de bande souple (560), la couche de transport de fluide (550) et l'enveloppe imperméable aux fluides (731) sont fixées entre la plaque supérieure (570) et la plaque de maintien (580), et le composant indicateur jetable (520) comprend en outre un coupleur (580) pour fixer de manière amovible le composant indicateur jetable (520) à un boîtier pour l'analyseur portatif (600).

4. Composant indicateur jetable (520) selon la revendication 3, dans lequel la plaque supérieure (570) et la plaque de maintien (580) sont agencées et conçues pour fournir un espacement prédéterminé pour recevoir des couches de composant indicateur ayant une capacité de transport de fluide prédéterminée vers la zone indicatrice (521).

5. Composant indicateur jetable (520) selon la revendication 2, contenu dans un emballage individuel.

6. Composant indicateur jetable (220) selon la revendication 1, le composant indicateur jetable (220) étant destiné à être utilisé dans un système de mesure de concentration d'analyte dans un fluide corporel, le composant indicateur jetable comprenant :
a) un coupleur (280) pour coupler le composant indicateur à un composant comportant au moins un spectrophotomètre (300) contenu dans un boîtier ;
b) une plaque supérieure (270) ;
c) une deuxième couche de bande souple (240) adjacente à l'enveloppe imperméable aux fluides ;
d) des moyens de fixation disposés sur la deuxième couche de bande souple (240) ; et
e) une plaque de maintien (280) ;
dans lequel :
I) la première couche de bande souple (260), la couche de transport de fluide (250), l'enveloppe imperméable aux fluides (431) et la deuxième couche de bande souple (240) sont empilées dans cet ordre et fixées entre la plaque supérieure (270) et la plaque de maintien (280) ;
et dans lequel la couche de transport de fluide est en communication fluidique avec la zone indicatrice.

7. Composant indicateur jetable (220) selon la revendication 6, dans lequel la plaque supérieure (270) et la plaque de maintien (280) sont agencées et conçues pour fournir un espacement prédéterminé pour recevoir des couches de composant indicateur ayant une capacité de transport de fluide prédéterminée vers la zone indicatrice (221).

8. Composant indicateur jetable (220) selon la revendication 6, dans lequel la couche de transport de fluide (250) comprend une barrière de fluide disposée entre des ouvertures dans l'enveloppe imperméable aux fluides (431).

9. Composant indicateur jetable (220) selon la revendication 6, comprenant une couche-culotte.

10. Composant indicateur jetable (220) selon la revendication 6, agencé et conçu pour une fixation amovible à une couche-culotte.

11. Composant indicateur jetable (220) selon la revendication 6, contenu dans un emballage individuel.
